# EUROPEAN PATENT APPLICATION

(11) **EP 3 168 958 A1**
(43) Date of publication of application: **17.05.2017**
(21) Application number: 16199160.9
(22) Date of filing: 16.11.2016
(51) Int. Cl.: H02J 7/00, F16M 11/04

(54) **PROGRAMMABLE POWER MODULE FOR MOBILE CART**

(30) Priority: 16.11.2015 GB 201520202
(71) Applicant: Bytec Healthcare Limited, Redhill, Surrey RH1 5DZ (GB)
(72) Inventor: Mifsud, Bernard, Redhill, Surrey RH1 5DZ (GB)
(74) Representative: Fry, David John

(57) **Abstract**

A mobile cart is disclosed, comprising a platform mounted above a wheeled based by a generally upright post and at least one battery docking unit on the post for the detachable mounting of a battery. A programmable control unit is associated with the at least one battery docking unit having a power output terminal at which a voltage is presented as the battery discharges. The control unit comprises a data bus, an integrated circuit DC-DC converter, and a programmable digital potentiometer connected to the DC-DC converter in such a way as to set its DC output voltage to a selected value in accordance with data received by the potentiometer from a remote computing device using the data bus, which selected output voltage is presented at the power output terminal in accordance with said data.

## Description

### Field of the Invention

This invention relates to a programmable power module for a mobile cart. More particularly, the invention relates to a programmable power module for providing a selected DC voltage for powering electrical equipment supported on the mobile cart.

### Background of the Invention

Mobile carts are used in many situations, one being in the medical field. For example, Applicant provides a range of mobile carts for use in the medical sector whereby the cart consists of a post mounted on a wheeled base with a platform on the top of the post on which electronic equipment is mounted, e.g. a computer terminal or hospital monitoring equipment. A power module is provided on the post, consisting of one or two battery docking stations for the detachable mounting of batteries. The batteries can be charged in situ or remote from the cart; when mounted on the docking station, the or each battery presents a voltage on an output terminal to which a lead can be connected to provide power to the mounted equipment on the platform. The cart can therefore be moved around a hospital environment without the need to use mains power.

The power module can include a controller providing intelligent operation, for example detecting when one docked battery has discharged below a particular level, and then switching to the other docked battery automatically to provide seamless power to the electronic equipment. This is sometimes termed 'hot swapping'. Additionally, the controller may authenticate the batteries before permitting discharge, e.g. to avoid inappropriate and potentially dangerous batteries from being used with the module.

A disadvantage exists in that the power module will be configured to deliver a predetermined d.c. voltage to the output terminal, which is typically factory set, e.g. 18 v, by means of hardware design within the module. If the end user wishes to use the cart with equipment requiring a different voltage, e.g. 24 v, then a separate DC-DC converter module is needed to convert the factory-set voltage to the new voltage. The converter module needs to be plugged to the output terminal of the power module, and the equipment to an output terminal of the converter. Also, the converter module is typically bulky. A different converter is required for each possible voltage variation. Overall, therefore, a cart will either be suitable only for certain equipment, or bulky and unsightly converters are needed to cater for different equipment.

### Summary of the Invention

A first aspect of the invention provides a mobile cart, comprising: a platform mounted above a wheeled based by a generally upright post; at least one battery docking unit on the post for the detachable mounting of a battery; and a programmable control unit associated with the at least one battery docking unit having a power output terminal at which a voltage is presented as the battery discharges, the control unit comprising a data bus, an integrated circuit DC-DC converter, and a programmable digital potentiometer connected to the DC-DC converter in such a way as to set its DC output voltage to a selected value in accordance with data received by the potentiometer from a remote computing device using the data bus, which selected output voltage is presented at the power output terminal in accordance with said data.

The DC-DC converter may be a single chip DC-DC converter, for example Picor Corporation's PI-3749. The digital potentiometer may be Analog Device's AD5292. Equivalents or variations thereof may be used.

The digital potentiometer may be configured as part of a voltage divider circuit at the output terminal(s) of the DC-DC converter.

The programmable control unit may comprise the DC-DC converter and digital potentiometer mounted on a single PCB. The programmable control unit may be housed within a common, sealed casing. The housing may be watertight, or substantially so. The housing may comprise a plurality of external terminals for connection to a detachable battery. The housing may comprises a connector for detachable mechanical connection to the post. The data bus may be an SPI bus or the like.

The programmable control unit may also comprise a processor or microcontroller arranged under program control to control one or more of the charging and discharging of a connected battery, authentication of a connected battery, data logging, and status data transmission.

A further aspect of the invention provides a programmable control unit associated with a battery or battery docking unit having a power output terminal at which a voltage is presented as the battery discharges, the control unit comprising a data bus, an integrated circuit DC-DC converter, and a programmable digital potentiometer connected to the DC-DC converter in such a way as to set its DC output voltage to a selected one of a plurality of discrete values in accordance with data received by the potentiometer from a remote computing device using the data bus, which selected output voltage is presented at the power output terminal in accordance with said data.

In preferred embodiments, the DC-DC converter is Picor Corporation's PI-3749 integrated circuit, or an equivalent or close variation thereof. The programmable digital potentiometer is preferably Analog Device's AD5292, or an equivalent or close variation thereof.

### Brief Description of the Drawings

The invention will now be described, by way of non-limiting example, with reference to the accompanying drawings in which:
Figure 1 is a perspective view of a medical trolley having a plurality of battery modules connected thereto via docking station(s);
Figure 2 is a perspective view of the docking station in Figure 1;
Figure 3 is a perspective view of a battery module connected to the docking station of Figure 2;
Figure 4 is a schematic, reverse plan view of metallic terminals of the battery module shown in Figure 3;
Figure 5 is a schematic system diagram of functional components of a power control module housed within the docking station of Figure 2;
Figure 6 is partial circuit schematic showing certain ones of the functional components of Figure 5; and

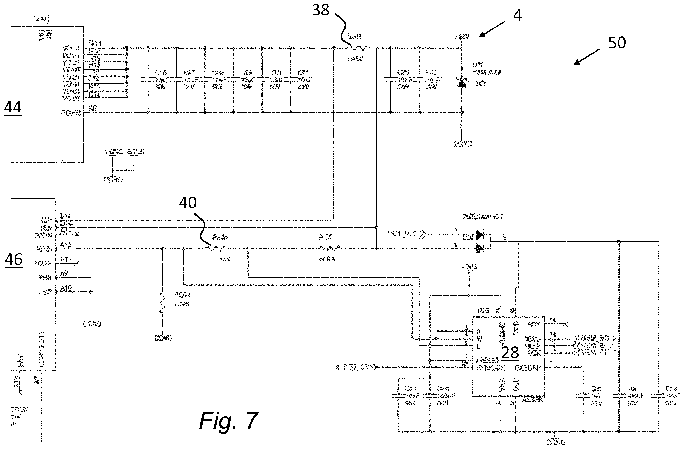

Figure 7 is a close-up view of part of the Figure 6 partial schematic.

### Detailed Description of Preferred Embodiments

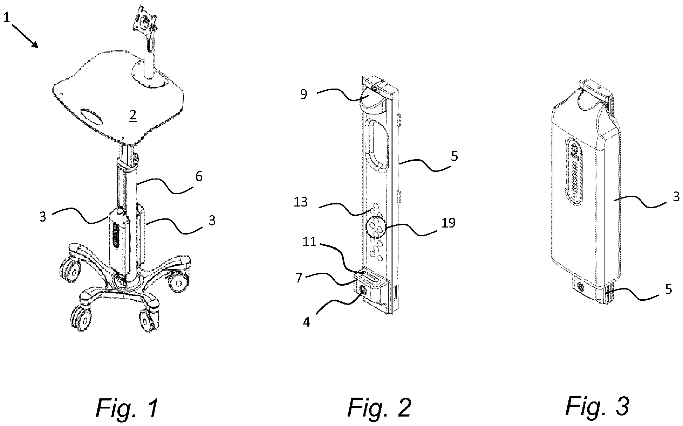

Figure 1 shows a medical cart or trolley 1 on which electronic equipment may be mounted and powered by a battery pack 3. The trolley 1 has a wheeled base.

In this case, first and second battery packs 3 are shown mounted on the upright post or stem 6 of the trolley 1. One battery pack 3 is connected to a docking station 5 (shown more clearly in Figure 2) to provide power to the electronic equipment, which connects to power outlet 4 using a lead, and the other is a replacement battery supported on the opposite side for discharge to the electronic equipment when the charge on the current battery pack is depleted. This so-called 'hot swapping' of the batteries 3 is handled by a processor or processors of a smart battery management system, hereafter termed 'power module' housed within the docking station 5.

Figure 2 shows the exterior of the docking station 5 which comprises an elongate unit having, at a lower end, a lower dock support 7 on which one end of the battery pack 3 is located in use and, at an upper end, a dock connector 9 into which the other end of the battery pack is releasably secured into position. In order to connect a battery pack 3 to the docking station 5, the lower end of the battery pack 3 is first located over a protruding part 11 of the lower dock support 7 and then the upper part is rotated towards the dock connector 9 where it is secured it in place. The power outlet 4 is located on the lower dock support 7, but can be provided anywhere on the docking station 5. It is in this case a female connector.

Figure 3 shows the battery pack 3 when secured to the docking station 5.

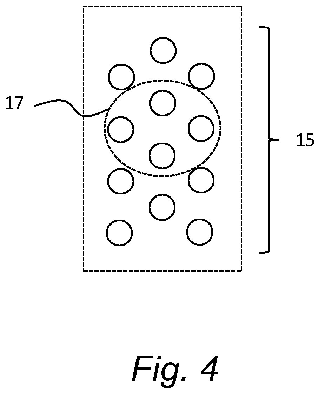

Referring back to Figure 2, the docking station 5 has a plurality of dome-like electrical terminals 13. Each is configured to connect with a corresponding terminal 15 provided on the rear face of a battery pack 3, for which see the schematic of Figure 4. In this example, four terminals 17 are used for the charging and discharge of electrical energy from the battery cell. These terminals are referred to as Vbat and the docking station has a corresponding set 19. Other terminals are used for different purposes, some of which will be explained below. The terminals 15 of the battery module 3 are preferably planar in form and recessed from the outer casing so that terminals cannot be accidentally shorted if placed on a flat metallic surface.

Although not essential to the current invention, to avoid damage to either the docking station 5 or the battery pack 3, the battery is required to be authenticated to the docking station. Prior to authentication, the docking station 5 isolates its Vbat terminals 19 to prevent the receiving or delivering of electrical energy for the purposes of charging the battery module 3 and the discharging of energy from the battery to electrical equipment on the trolley 1, i.e. the load. Only when a connected battery pack 3 is authenticated is the isolation removed and charging and discharge permitted. The primary purpose of this is to avoid incompatible batteries (or batteries that have not gone through the necessary quality control) from damaging the docking station 5 or from becoming hazardous.

The above authentication functionality is an example of intelligent power control, which is handled by the power module circuitry on a single PCB housed within the docking station 5, which has a neat and compact design. A microprocessor or controller works under program control to manage a variety of functions relating to operation of the docking station 5, including the above-described hot-swapping function whereby discharge of energy from a depleted battery 3 is switched to the other battery which has greater charge, as well as the battery authentication. Associated with the microprocessor or controller is memory storing firmware, and/or one or more programs, as well as volatile and non-volatile memory for the storage of data. The microprocessor is connected to a data bus, which may be a Serial Peripheral interface (SPI) bus, to which an external computer terminal such as a PC or tablet computer can connect through a USB connector or the like to read and write data. In terms of reading data, this may be useful for diagnostic purposes. In terms of writing data, this is used for updating firmware, software and making certain modifications to the operation of the docking station, as will be explained below.

Referring to Figure 4, components providing a power module 20 are provided on a PCB housed within the docking station 5.

A controller 22 is connected to a dual battery system manager, a DC to DC converter 26, a digital potentiometer 28, memory 30, and a SPI data bus 32. The controller 22 may be a microprocessor, plural microprocessors, a microcontroller or plural microcontrollers. The controller 22 may be connected to other modules also.

The controller 22 works under the control of firmware or software stored on memory 30 to control the overall operation of the docking station 5, including some aspects of power control. This firmware or software can be updated via the SPI data bus 32 using an external computer terminal 36 which connects to the interface 34, e.g. using a USB lead or through a wireless protocol such as Bluetooth or WiFi. RAM (not shown) may be associated with the controller 22 for the temporary storage of data.

The dual battery system manager24 is an integrated circuit system management bus (SMBus) battery charger and selector controller configured to handle charging and selection of dual smart battery systems connected via terminals 13, as in the present case. A known IC is LTC1760 in which three SMBus interfaces allow the IC to servo to the internal voltage and currents measured by the batteries 3 while allowing an SMBus host device to monitor either's status. Switching between the batteries 3 is seamless. The controller 22 can communicate requests to the dual battery system manager 24 and receive status data back from it. For example, the controller 22 can receive data from a battery 3 for checking the authenticity of the battery against pre-stored authentication data. Thereafter, the controller 22 can control the battery system manager 24 to discharge energy from the current battery 3, e.g. to prevent discharge in the event that the battery is not authenticated and/or enable discharge only when enabled. The controller 22 can receive information from the battery system manager 24 as to other issues.

The dual battery system manager 24 is connected to the DC to DC converter 26 so that discharging electrical energy from a battery 3 is converted to the required d.c. voltage to be presented at the output terminal 4.

As noted in the background, typically the DC to DC converting stage is a factory-set module, i.e. set to a particular voltage, e.g. 18 v, by means of various soldered components on the PCB which is sealed within the docking station 5 casing.

In this embodiment, the power module 20 within docking station 5 is programmable through interface 24 and the SPI bus 32 to set the d.c. voltage presented at output terminal 4 without having to physically change the PCB or indeed open the docking station casing. Further, no unsightly adaptors are required for further DC-DC conversion.

To provide this, the DC to DC converter 26 is a single-chip IC, in this case Picor Corporation's PI-3749. This IC is a high-efficiency, wide range DC-DC converter and zero voltage switching buck-boost regulator. It comprises an internal controller, power switches, and support components, requiring minimal external components to provide the full functionality. Its provision on a single chip minimises also the footprint required on the PCB and can be combined with the other hardware indicated as the power module 20.

Rather than using physical resistor components to set the particular output voltage for presenting to the output terminal 4, the programmable digital potentiometer 28 is connected at the appropriate output pins of the DC to DC converter 26 as part of a voltage divider.

In this embodiment, the programmable digital potentiometer 28 is Analog Device's AD5292, which is a single-channel, 256/1024 position digital potentiometer combining variable resistor performance with non-volatile memory in a compact IC package. The twenty time programmable (TP) memory enables unlimited adjustments to resistor values prior to programming, which sets the resistance value by freezing the wiper position, and there are twenty opportunities for permanent programming, which is performed through the SPI bus 32 from an external device 36. Again, the IC is a single chip, provides stability and accuracy over discrete mechanical potentiometers, and minimises the footprint required enabling all functionality on the PCB within the casing.

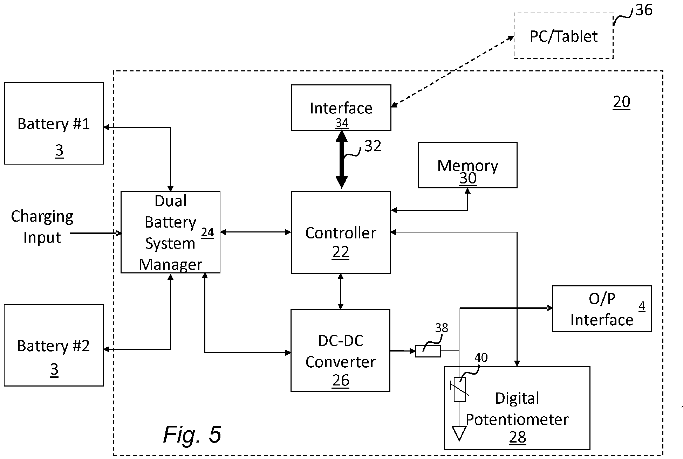

Thus, the power module 20 can have its output voltage set at a required value simply by programming the digital potentiometer 28 to the required resistance according to the voltage divider formula, which is readily understood. The voltage divider is indicated in Figure 5 by a resistor 38 of fixed value between the DC to DC converter 26 and the variable resistor 40 within the digital potentiometer 28.

Programming of the potentiometer 28 is performed by an external computer terminal 36 running appropriate software, e.g. a dedicated program or 'app', enabling both the factory and possibly even the end-customer to customise the output voltage at output terminal 4 without having to open the casing of the docking station 4. A customer can simply inform the manufacturer of their voltage requirement, e.g. 24v, and the manufacturer then sets the digital potentiometer 28 to the appropriate resistor value from the default (e.g. for 18v) prior to supply. This external programming is particularly advantageous in the field of medical carts, whereby circuitry such as that within the docking station 5 is sealed, usually watertight, to enable regular cleaning and disinfecting, and also because it is susceptible to liquid spillage in use.

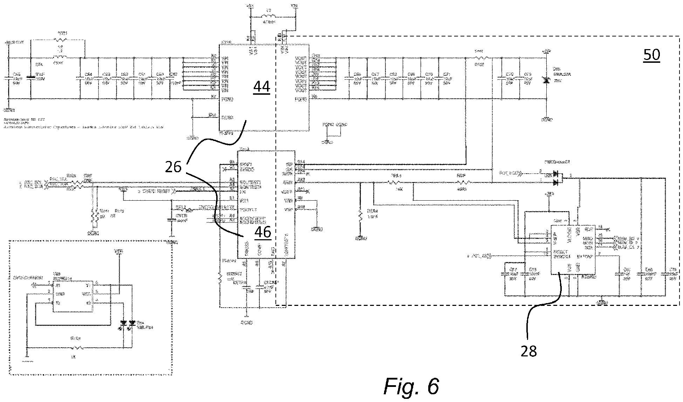

Figure 5 is a circuit schematic of part of the power module 20 in docking station 5, including the DC to DC converter 26 (divided into two functional components 44, 46 for ease of explanation) and the digital potentiometer 28. Figure 6 shows a subset 50 of the Figure 5 schematic in close-up, where it will be seen that the variable resistor 40 is indicated within the schematic relative to R1 in the voltage divider to provide a programmable means of setting Vout at terminal 4.

Whilst the power module 20 in this case comprises a set of components mounted on a PCB within a battery docking station 5 on a medical cart 1, the module could be provided in other hardware forms for other applications. For example, the application of the DC to DC converter 26 and digital potentiometer 28, in association with a data bus for external programming, could be used within any cased module utilising battery power from a detachable battery, thereby enabling repeated adjustment of the output voltage through programming rather than opening the casing. The power, voltage and current ranges are not limited to those mentioned above, whereby ranges from 12 to 28 v at 150 w or 200 w have been utilised. Whilst a SPI bus 32 and SPI interface 34 have been used as examples, it will be appreciated that other forms of bus can be used for the programming.

It will be appreciated that the above described embodiments are purely illustrative and are not limiting on the scope of the invention. Other variations and modifications will be apparent to persons skilled in the art upon reading the present application.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

## Claims

1. A mobile cart, comprising:
a platform mounted above a wheeled based by a generally upright post;
at least one battery docking unit on the post for the detachable mounting of a battery; and
a programmable control unit associated with the at least one battery docking unit having a power output terminal at which a voltage is presented as the battery discharges, the control unit comprising a data bus, an integrated circuit DC-DC converter, and a programmable digital potentiometer connected to the DC-DC converter in such a way as to set its DC output voltage to a selected value in accordance with data received by the potentiometer from a remote computing device using the data bus, which selected output voltage is presented at the power output terminal in accordance with said data.

2. The mobile cart according to claim 1, wherein the DC-DC converter is a single chip DC-DC converter.

3. The mobile cart according to claim 2, wherein the DC-DC converter is Picor Corporation's PI-3749.

4. The mobile cart according to any preceding claim, wherein the digital potentiometer is Analog Device's AD5292.

5. The mobile cart according to any preceding claim, wherein the digital potentiometer is configured as part of a voltage divider circuit at the output terminal(s) of the DC-DC converter.

6. The mobile cart according to any preceding claim, wherein the programmable control unit comprises the DC-DC converter and digital potentiometer mounted on a single PCB.

7. The mobile cart according to claim 6, wherein the programmable control unit is housed within a common, sealed casing.

8. The mobile cart according to claim 7, wherein the casing is watertight, or substantially so.

9. The mobile cart according to claim 7 or claim 8, wherein the casing comprises a plurality of external terminals for connection to a detachable battery.

10. The mobile cart according to any of claims 7 to 9, wherein the casing comprises a connector for detachable mechanical connection to the post.

11. The mobile cart according to any preceding claim, wherein the data bus is a SPI bus or the like.

12. The mobile cart according to any preceding claim, wherein the programmable control unit further comprises a processor or microcontroller arranged under program control to control one or more of the charging and discharging of a connected battery, authentication of a connected battery, data logging, and status data transmission.

13. A programmable control unit associated with a battery or battery docking unit having a power output terminal at which a voltage is presented as the battery discharges, the control unit comprising a data bus, an integrated circuit DC-DC converter, and a programmable digital potentiometer connected to the DC-DC converter in such a way as to set its DC output voltage to a selected one of a plurality of discrete values in accordance with data received by the potentiometer from a remote computing device using the data bus, which selected output voltage is presented at the power output terminal in accordance with said data.
